# EUROPEAN PATENT SPECIFICATION

(11) **EP 2 117 355 B1**
(45) Date of publication and mention of the grant of the patent: **26.04.2017**
(21) Application number: 07747346.0
(22) Date of filing: 13.03.2007
(51) Int. Cl.: A61K 31/702, A61K 31/715, A61K 35/74, A61P 43/00

(54) **METHOD OF IMPROVING SKILLS WITH A COMPOSITION COMPRISING NON-DIGESTIBLE SACCHARIDE**
VERFAHREN ZUR VERBESSERUNG VON FÄHIGKEITEN MIT HILFE EINER ZUBEREITUNG ENTHALTEND NICHTVERDAULICHE SACCHARIDE
PROCÉDÉ PERMETTANT D'AMÉLIORER DES APTITUDES AVEC UNE COMPOSITION COMPRENANT UN SACCHARIDE NON DIGESTIBLE

(43) Date of publication of application: 18.11.2009
(73) Proprietor: N.V. Nutricia, 2712 HM Zoetermeer (NL)
(72) Inventor: STAHL, Bernd, D-61191 Rosbach-Rodheim (DE); KNOL, Jan, NL-6708 SM Wageningen (NL)
(74) Representative: Nederlandsch Octrooibureau
(86) International application number: PCT/NL2007/050100
(87) International publication number: WO 2008/111832

(56) References cited:
- WO-A-96/31219
- WO-A-2006/091103
- ANONYMOUS: "Aptamil" INTERNET CITATION, [Online] 16 January 2004 (2004-01-16), XP002412131 Retrieved from the Internet: URL:http://www.milupa.ch/images/prebiotica _pressemitteilung.pdf> [retrieved on 2006-12-19]
- MORISHITA Y ET AL: "Galactooligosaccharide in combination with Bifidobacterium and Bacteroides affects the population of Clostridium perfringens in the intestine of gnotobiotic mice" NUTRITION RESEARCH, vol. 22, no. 11, November 2002 (2002-11), pages 1333-1341, XP002295913 ISSN: 0271-5317
- FINEGOLD SYDNEY M ET AL: "Gastrointestinal microflora studies in late-onset autism" CLINICAL INFECTIOUS DISEASES, vol. 35, no. Supplement 1, 1 September 2002 (2002-09-01), pages S6-S16, XP009093350 ISSN: 1058-4838
- SANDLER RICHARD H ET AL: "Short-term benefit from oral vancomycin treatment of regressive-onset autism" JOURNAL OF CHILD NEUROLOGY, vol. 15, no. 7, July 2000 (2000-07), pages 429-435, XP009093351 ISSN: 0883-0738
- BOLTE E.R.: "Autism and Clostridium tetani", MEDICAL HYPOTHESES, vol. 51, 1998, pages 133-144,

## Description

### FIELD OF THE INVENTION

The present invention relates to a therapy aimed at improving language and/or social skills in infants through administration of components stimulating the development of a healthy intestinal flora.

### BACKGROUND

A good development of an infant both in language skills and social skills is very important to all parents. The speed and direction of development of a human in these area is multifactorial. Significantly impaired development of language social and/or communication skills are signs of diseases such as Asperger syndrome and Pervasive Developmental Disorder.

Considerable amounts of research are done to unravel the methods to stimulate an optimal development of the infant. It is an aim of many parents to provide the infant with optimal chances later in life by for example stimulating an optimal brain development or stimulate the immune system for prevention of diseases, as described for example in WO 2005039597. Stimulating visual acuity and brain development is for example according to US 6,036,992 stimulated by a specific blend of long chain polyunsaturated fatty acids.

### SUMMARY OF THE INVENTION

The present inventors recognized that an important cause of the development of social skills and language skills coincides with the occurrence of an abnormal intestinal flora microbiota (flora). Overgrowth of potentially pathogenic micoroorganosms, e.g. bacteria and/or yeasts, particularly Clostridia and/or candida, are an important cause of an impaired development of those skills, even to an extent where it can be categorized as a disease (e.g. Asperger syndrome or Pervasive Developmental Disorder, Not Otherwise Specified (PPD-NOS).

Sandler et al, 2000 (J Child Neurol 15(7):429-435) describes that in a pilot study of oral vancomycin therapy (antibiotic) in autistic children, significant cognitive improvement was reported following vancomycin treatment. Approximately a week after the course of antibiotics had finished, significant regression was observed in autistic symptoms. Hence, the flora seems to play a role in the autism. Additionally, the fecal flora of children with regressive autism have higher clostridial counts (Bingham et al, 2003, Food Sc Techn Bull: Functional Foods 1(4) 1-11; Finegold SM et al, 2002, Clin Infect Dis. 35 (Suppl 1):S6-S16)..

Bolte et al. 1998 (Medical Hypotheses 51:133-144) report that some children with autism show a significant reduction in stereotyped behaviours when treated with antimicrobials effective against intestinal clostridia.

Without wishing to be bound by theory, it is the present inventors believe that the potentially pathogenic bacteria (particularly Clostridium) produce toxins which are transported from the intestine to the brain. These toxins have a negative effect on the language skills, communication skills, social skills and/or reading skills, which after long exposure to the toxins can even cause irreversible damage.

Hence the present invention particularly aims to reduce potentially pathogenic micro-organisms (particularly a high *Clostridium* counts), thereby stimulating the development and/or preventing regression of language skills, communication skills, social skills and/or reading skills.

The present inventors have found that by reducing the overgrowth of potentially pathogenic bacteria, particularly Clostridia, the naturally occurring microorganisms in the intestinal flora, in particular Lactobacilli and Bifidobacteria, are significantly stimulated. The present inventors have found that the overgrowth by potentially pathogenic micro-organisms can be reduced by oral administration of non-digestible saccharides, resulting in an improved development of language skills.

As the present invention particularly aims to stimulate the development of language skills, communication skills, social skills, reading skills by reducing the *Clostridium* counts, the present invention can be advantageously used in (sub)populations suffering from such high *Clostridium* overgrowth. In a preferred embodiment, the present method is particularly suitable for babies delivered via cesarean section and/or premature babies. Babies born via cesarean section have a higher percentage of *Clostridium* and have an increased risk of developing autism (Hultman et al. Epidemiology 2002, 13(4):417-23). Hence, reduction of *Clostridium* counts and/or stimulation of language skills, communication skills, social skills and/or reading skills in this subpopulation is particularly desirable.

Additionally the present method is advantageously suitable for infants suffering from an autistic spectrum disorder receiving an antibiotic treatment. While receiving antibiotics, and Clostridia counts are decreased, cognitive improvement in these infants is significant. However, shortly after the antibiotic treatment is terminated, a regression takes place. Hence, in one embodiment the present invention relates to preventing regression of cognitive functions after antibiotic treatment in patients suffering from autistic spectrum disorder.

A main advantage of the present invention is that the non-digestible saccharides can be easily included into nutritional compositions. Nutritional compositions are easily administered to infants. For young infants the non-digestible saccharides and bacteria can for example be included in the infant nutrition formula or added to breast milk.

In a press release made public on 16 January 2004 and retrieved from http://www.milupa.ch/images/prebiotica_pressemitteilung.pdf, Milupa disclosed that an infant nutrition comprising indigestible saccharides can be used to improve the intestinal flora of an infant.

WO 2006/091103 describes an infant nutrition containing non-digestable saccharides to equalise the *Bifidobacteria* species distribution in the gastrointestinal tract.

Morishita et al. 2002 (Nutrition Research 22:1333-1341) describe that administering GOS results in a change in intestinal microflora. *Bifidobacterium breve* markedly increases and *Clostridium perfrigens* decreases.

WO 96/31219 describes a method of inhibiting *Clostridium difficile* infection by enterally administering indigestible oligosaccharide.

### DETAILED DESCRIPTION OF PREFFERED EMBODIMENTS

The present invention concerns the use of a composition comprising non-digestible saccharide for the manufacture of a composition for preventing a decline in or improving one or more of (i) language skills, (ii) communication skills, (iii) social skills and/or (iv) reading skills. Also the invention may be worded as use of a composition comprising non-digestible saccharide for preventing a decline in or improvement of one or more of (i) language skills, (ii) communication skills, (iii) social skills and/or (iv) reading skills.

### Non-digestible saccharides

The non-digestible saccharide in the present composition preferably is capable of preventing overgrowth of pathogenic microorganisms by (i) being fermentable into organic acids (preferably lactic acid, butyrate, propionate and/or acetate) and/or stimulating the growth of lactobacilli and/or bifidobacteria and/or (ii) being capable of reducing the adherence of the potentially pathogenic organisms to the intestinal epithelium, thereby reducing the overgrowth of these pathogenic microbes. The present saccharide preferably stimulates the growth or relative percentage of *Bifidobacteria* and/or *Lactobacilli.*

Preferably the present composition comprises at least one non-digestible oligosaccharide selected from the group consisting of fructo-oligosaccharides (including inulins), non-digestible dextrins, galacto-oligosaccharides (including transgalacto-oligosaccharides), xylo-oligosaccharides, arabino-oligosaccharides, arabinogalacto-oligosaccharides, gluco-oligosaccharides (including cyclodextrins and gentio-oligosaccharides), chito-oligosaccharides, glucomanno-oligosaccharides, galactomanno-oligosaccharides, mannan-oligosaccharides, fuco-oligosaccharides, galacturonic acid oligosaccharides, guluronic acid oligosaccharides, mannuronic acid oligosaccharides, iduronic acid oligosaccharides, riburonic acid oligosaccharides, glucuronic acid oligosaccharides and mixtures thereof, more preferably fructo-oligosaccharides, galacto-oligosaccharides (including transgalacto-oligosaccharides), sialyllyctoses (3SL, 6SL), fucosyllactoses (2FL, 3FL), galactosyllactoses, lacto-N-tetraose (LNT), lacto-N-neotetraose (neo-LNT), fuco-oligosaccharides and galacturonic acid oligosaccharides and mixtures thereof. More preferably present composition comprises at least one non-digestible oligosaccharide selected from the group consisting of fructo-oligosaccharides (including inulins), galacto-oligosaccharides (including transgalacto-oligosaccharides), fuco-oligosaccharides, galacturonic acid oligosaccharides and mixtures thereof More preferably present composition preferably comprises galacto-oligosaccharides, more preferably galactooligosaccharides and fructooligosaccharides, more preferably (i) galacto-oligosaccharides, (ii) fructo-oligosaccharides and (iii) galacturonic acid oligosaccharides. Also of interest are gangliosides, such as for example at least one selected from the group consisting of GM3 and GD3. The preferred mixtures are particularly effective, preferably synergistic, in preventing or reducing overgrowth of potential pathogens by an optimal stimulation of the *Bifidobacteria* and/or *Lactobacilli* and reduced adherence of pathogenic organisms such as *Clostridium.*

Stimulation of the colonization or relative proportion of lactic acid producing bacteria can be achieved by administration of a non-digestible saccharide with relatively low degree of polymerization (DP). Advantageously, the present composition comprises non-digestible saccharides preferably with a degree of polymerization (DP) of 2 to 250, more preferably 2 to 100, more preferably 2 to 10. The present composition preferably comprises at least 50 wt.% non-digestible oligosaccharides with a degree of polymerization of 2 to 60 based on total weight of oligosaccharides with DP 2 to 250. The present composition preferably comprises (i) galacto-oligosaccharides with a DP of 2 to 10 and/or fructo-oligosaccharides with a DP of 2 to 60.

The present non-digestible saccharides are preferably (i) not or only partially digested in the intestine by the action of acids or digestive enzymes present in the human upper digestive tract (small intestine and stomach) and (ii) fermented by the human intestinal flora. For example, sucrose, lactose, maltose and the maltodextrins are considered digestible. In one embodiment the present non-digestible saccharide is a soluble, indigestible, fermentable saccharide. In one embodiment the present non-digestible saccharide is selected from the group consisting of galactooligosaccharides, fructooligosaccharides and fructopolysaccharides.

The present method preferably comprises the administration of a serving comprising between 0.05 and 25 grams non-digestible saccharide, preferably between 0.1 and 5 grams. The present method preferably comprises the administration of a serving comprising between 0.05 and 25 grams galacto-oligosaccharides, preferably between 0.1 and 5 gram galacto-oligosaccharides. The present method preferably comprises the administration of 0.05 to 25 grams non-digestible saccharide per day, preferably between 0.1 and 5 grams per day. The present method preferably comprises the administration between 0.05 and 25 grams galacto-oligosaccharides per day, preferably between 0.1 and 5 gram galacto-oligosaccharides per day.

### Galacturonic acid oligosaccharide

In a preferred embodiment the present composition comprises galacturonic acid oligosaccharides. The galacturonic acid oligosaccharides of the invention advantageously reduce the adhesion of (potentially) pathogenic micro-organisms to the intestinal epithelial cells, thereby reducing colonization of (nosocomial) pathogenic microbes in the colon of the infant. Furthermore, the galacturonic acid oligosaccharides of the present invention stimulate the formation of a healthy intestinal flora and are fermented in the lower part of the gastro-intestinal tract, resulting in a production of intestinal organic acids and a reduction of intestinal pH, which inhibit the growth of (potentially) pathogenic microoraganisms. Due to the reduced adhesion and/or colonization, translocation of toxins produced by certain Clostridium species is reduced. The term galacturonic acid oligosaccharide as used in the present invention preferably refers to an oligosaccharide wherein at least 50% of the monosaccharide units present in the oligosaccharide are galacturonic acid. The present composition preferably comprises galacturonic acid oligosaccharide with a DP between 2 and 250, preferably 2 and 50, more preferably 2 and 20. The galacturonic acid oligosaccharides used in the invention are preferably prepared from pectin, pectate, and/or polygalacturonic acid. The galacturonic acid oligosaccharide is preferably derived from pectin, i.e. a pectin oligosaccharide. Preferably the pectin oligosaccharide is preferably prepared by hydrolysis and/or beta-elimination of fruit pectin and/or vegetable pectin, more preferably from apple, citrus and/or sugar beet pectin, more preferably the apple, citrus and/or sugar beet pectin has been treated by at least a lyase. The preparation of the pectin product may also include pH treatment and/or temperature treatment and/or pressure treatment. Preferably the present composition contains a pectin degradation product, preferably with a DP of 2 to 250.
In a preferred embodiment, at least one of the terminal hexuronic acid units of the galacturonic acid oligosaccharide has a double bond, which is preferably situated between the C₄ and C₅ position of the terminal hexuronic acid unit. The double bond effectively protects against attachment of pathogenic bacteria to intestinal epithelial cells.

The present composition preferably comprises between 0.01 and 10 grams galacturonic acid oligosaccharide with a DP of 2 to 250 per 100 gram dry weight of the composition, more preferably between 0.05 and 6 gram, even more preferably 0.2 to 2 gram. Preferably the present composition comprises between 0.01 and 10 grams galacturonic acid oligosaccharide with a DP of 2 to 25 per 100 gram dry weight of the nutritional composition, more preferably between 0.05 and 6 gram, even more preferably 0.2 to 2 gram. The short chain galacturonic acid oligosaccharides are more effective and/or better suitable for inclusion in the present composition. Preferably the present composition contains of (i) galactooligosaccharides (ii) fructopolysaccharides and/or fructooligosaccharides and (iii) galacturonic acid oligosaccharide.

### Lactic acid producing bacteria

The present composition preferably comprises lactic acid producing bacteria, either living or dead. Dead bacteria can for example be prepared by inactivating living bacteria by heat treatment and/or sonication. Living lactic acid bacteria are advantageously administered to stimulate fast colonization of the infant's intestinal tract, thereby preventing or reducing overgrowth of (potentially) pathogenic organisms such as Clostridium. Lactic acid producing bacteria are preferably provided as a mono- or mixed culture of live microorganisms. The present composition preferably comprises 10² to 10¹³ colony forming units (cfu) of (lactic acid producing) bacteria per gram dry weight of the present composition, preferably 10² to 10¹² cfu, more preferably 10⁵ to 10¹⁰ cfu, most preferably from 10⁴ to 5x10⁹ cfu.

Preferably the present composition comprises bacteria of the genus *Lactobacillus* and/or *Bifidobacterium.* Preferably the composition comprises at least one *Bifidobacterium* selected from the group consisting of *B. longum, B. breve, B. infantis, B. catenulatum, B. pseudocatenulatum, B. adolescentis, B. animalis, B. gallicum, B. lactis* and *B. bifidum,* more preferably at least one *Bifidobacterium* selected from the group consisting of *B. breve, B. infantis, B. bifidum, B. catenulatum, B. longum,* even more preferably at least one *Bifidobacterium* selected from the group consisting of *B. breve* and *B. longum,* most preferably *B. breve.*
Preferably the present composition contains a *Lactobacillus* selected from the group consisting of *L. casei, L. reuteri, L paracasei, L. rhamnosus, L. acidophilus, L. johnsonii, L. lactis, L. salivarius, L. crispatus, L. gasseri, L. zeae, L. fermentum* and *L. plantarum,* more preferably *L. casei, L. paracasei, L. rhamnosus, L. johnsonii, L. acidophilus, L. fermentum* and most preferably *L. paracasei.* Even more preferably the present composition comprises *Bifidobacterium* and *Lactobacillus more preferably Bifidobacterium breve* and *Lactobacillus paracasei.*

### Application

The present invention provides a composition for use in the improvement of one of more of (i) language skills; (ii) communication skills; (iii) social skills and/or (iv) reading skills. As the present method is particularly useful in early life, the present composition is preferably administered to children with the age between 0 and 10 years, more preferably to infants with the age between 0 and 3 years. The improvement of skills may be communicated to the consumer by wording such as "support development of language skills" or visually by a picture indicating the support or improvement. In young infants, the intestinal flora needs to be established and hence it is particularly important to prevent Clostridium overgrowth in these infants.

Assessment of limitations and need for improvement of language skills, communication skills and social skills can be suitably done by specialized medical personnel, such as a speech-language pathologist. (see for example Assessing speech, language and communication, Marans et al, 1999 (Comp Psy Assess of Young Children , Vol 8 (2):297321). Language skills include particularly receptive and expressive language skills, phonetic repertoire, articulation and vocabulary. The communication skills are often intimately related to verbal performance and reading skills (see for example Developmental pathways through childhood for children treated in a neonatal intensive care unit by Ellison P et al (1992); Vol 81 (suppl 380)1-13). Methods for determining the stages and development of these skills stage of a human, particularly of an infant are known in the art.

The present composition is particularly suitable for the subpopulation, particularly infants, suffering from *Clostridium* overgrowth, particularly infants delivered via cesarean section and/or premature infants and/or an infants suffering from Asperger syndrome and PPD-NOS.

Furthermore, the present invention can be suitably used to prevent regression due to the pathogenic recolonisation of the intestine following antibiotic treatment. Hence the present invention provides the treatment and/or prevention of an autistic spectrum disorder in an infant having received an antibiotic treatment, said method comprising administering the present non-digestible saccharide. In one aspect, the present invention provides for administering the present non-digestible saccharide after the treatment with antibiotics to an infant suffering from autistic spectrum disorder. In still a further aspect the present invention provides the prevention of a disorder selected from Asperger syndrome and PPD-NOS.

As reducing the *Clostridium* counts in the (childs) intestine is particularly relevant, the present invention provides a method for reducing the *Clostridium* counts in (i) an infant delivered via cesarean section, (ii) a premature infant, (iii) an infant suffering from a autistic spectrum disorder and/or (iv) an infant having been subjected to antibiotic treatment, said method comprising administering the present non-digestible saccharide.

### Formulae

The present composition is preferably enterally administered, more preferably orally. The present composition is preferably an infant formula. The present composition is preferably a synthetic formula, prepared by admixing different ingredients. The present composition is not a naturally (non-treated) occurring mammalian milk, e.g. not human breast milk. The present composition can be advantageously used as a complete nutrition for infants. The present composition preferably comprises lipid, protein and carbohydrate and is preferably administered as a liquid food. The term "liquid food" as used in the present invention includes dry food (e.g. powders) accompanied with instructions so as to admix said dry food mixture with a suitable liquid (e.g. water).

The present composition preferably provides nutrition to the infant, and comprises a lipid component, a protein component and a carbohydrate component. The lipid component preferably provides 5 to 50% of the total calories, the protein component preferably provides 5 to 50% of the total calories, and the carbohydrate component preferably provides 15 to 90% of the total calories. The present composition is preferably used as an infant formula, wherein the lipid component provides 35 to 50% of the total calories, the protein component provides 7.5 to 12.5% of the total calories, and the carbohydrate component provides 40 to 55% of the total calories. For calculation of the % of total calories for the protein component, the total of energy provided by the proteins, peptides and amino acids needs to be taken.

The protein component used in the present composition preferably comprises at least one selected from the group consisting of non-human animal proteins (such as milk proteins, meat proteins and egg proteins), vegetable proteins (such as soy protein, wheat protein, rice protein, potato protein and pea protein), free amino acids and mixtures thereof. Cow's milk derived nitrogen source, particularly cow milk proteins such as casein and whey proteins are particularly preferred. Preferably the present composition has a relatively low protein content. Excess proteins are not digested in the upper part of the gastrointestinal tract and reach the colon, where these excess protein are (partially) converted in toxins by certain species of Clostridium. Hence, a low protein content is desirable and improves the present composition for use according to the present invention. The present composition preferably less than 20 g protein per 100 g dry weight of the composition, preferably less than 15 g, more preferably less than 12 g. In many occasions protein is however essential for growth (e.g. for infant nutrition. Hence the present composition preferably comprises at least 5 g protein per 100 g dry weight of the composition, preferably at least 7 g, most preferably at least 10 g.
Preferably the composition comprises digestible carbohydrates. The digestible carbohydrates used in the present composition are preferably selected from the group consisting of sucrose, lactose, glucose, fructose, corn syrup solids, starch and maltodextrins, and mixtures thereof, more preferably lactose. The present composition preferably has an osmolality between 50 and 500 mOsm/kg, more preferably between 100 and 400 mOsm/kg. In view of the above, it is also important that the liquid food does not have an excessive caloric density, however still provides sufficient calories to feed the subject. Hence, the liquid food preferably has a caloric density between 0.1 and 2.5 kcal/ml, even more preferably a caloric density of between 0.5 and 1.5 kcal/ml, most preferably between 0.6 and 0.8 kcal/ml.

The verb "to comprise" as is used in this description and in the claims and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there is one and only one of the elements. The indefinite article "a" or "an" thus usually means "at least one".

### EXAMPLES

### Example 1

In order to determine the effect on intestinal flora, an prebiotic containing infant formula (PF) with fructo- and galactooligosaccharides (FOS/GOS) was compared with a standard formula (SF). The microflora was determined using fluorescence *in situ* hybridization (FISH).

At term born, healthy, bottle-fed infants were enrolled within 0-14 days after birth and received either PF or SF. Fecal samples were taken at enrollment and at the age of 6 weeks. Using specific probes Bifodobacterium (Bif164) and Clostridium (Chis150/Clit135) were determined.

Enumeration of the feacal flora using FISH showed significant changes in the microflora. In parallel to the increase in Bifidobacteria the proportion of Clostridia was reduced in the group fed PF.

| | **Proportion of total bacteria** | | |
|---|---|---|---|
| **Infant formula** | | **Bif164** | **Chis150/Clit135** |
| **SF** | 0-2 weeks | 32.3 | 7.67 |
| | 6 weeks | 33.9 | 8.54 |
| **PF** | 0-2 weeks | 36.2 | 3.75^{#} |
| | 6 weeks | 68.7^{*#} | 2.30^{#} |
| *p<0.05 6wks vs. 0-2 wks; ^{#}p<0.05 PF vs. SF | | | |

An infant formula containing prebiotic oligosaccharides (FOS/GOS) reduces the level of pathogenic Clostridium bacteria and thus is indicative of a positive effect on language skills, communication skills, social skills and/or reading skills.

### Example 2

Packaged infant milk formula provided with a label indicating that the formula can be suitably used to improve communication abilities; the formula containing per 100 ml final product (and per 13.1 g powder):

| | |
|---|---|
| 8 energy % protein | 1.4 g (casein whey mixture) |
| 45 energy % digestible carbohydrates | 7.5 g |
| 47 energy % fat | 3.5 g |
| transgalactooligosaccharides (TOS) | 0.3 g |

### Example 3:

Packaged infant milk formula according to example 1, further containing per 100 ml final product (and per 13.1 g powder);

| | |
|---|---|
| Raftilin HP, Orafti BE) | 0.1 g |
| tuna fish oil | 0.3 gram |
| 40% arachidonic acid oil | 0.3 gram |
| (DSM Food Specialties, Delft, Netherlands) | |

### Example 4:

Packaged infant milk formula according to example 1 or 2, wherein the packaging is provided with a label indicating that the formula can be suitably used to improve social skills.

### Example 5:

Packaged infant milk formula according to example 1, wherein the packaging is provided with a label indicating that the formula can be suitably used to improve language and reading skills.

## Claims

1. Use of a composition comprising non-digestible oligosaccharide, wherein the non-digestible oligosaccharide comprises galacto-oligosaccharides and fructo-oligosaccharides, for the manufacture of a composition for preventing a decline in or improving one or more of (i) language skills, (ii) communication skills, (iii) social skills and/or (iv) reading skills.

2. Use according to claim 1 wherein the non-digestible oligosaccharide further comprises galacturonic acid oligosaccharides.

3. Use according to claims 1 or 2, wherein the composition is a nutritional composition wherein the lipid component provides 35 to 50% of the total calories, the protein component provides 7.5 to 12.5% of the total calories, and the carbohydrate component provides 40 to 55% of the total calories.

4. Use according to any one of the preceding claims, wherein the composition is administered to an infant.

5. Use according to claim 4, wherein the infant is delivered via caesarean section and/or is a premature infant.

## Patentansprüche

1. Verwendung einer Zusammensetzung umfassend nicht-verdauliches Oligosaccharid, wobei das nicht-verdauliche Oligosaccharid Galacto-Oligosaccharide und Fructo-Oligosaccharide umfasst, für die Herstellung eines Zusammensetzung zur Verhinderung einer Abnahme oder zur Verbesserung einer oder mehrerer von (i) Sprachfertigkeiten, (ii) Kommunikationsfertigkeiten, (iii) sozialen Fertigkeiten und/oder (iv) Lesefertigkeiten.

2. Verwendung nach Anspruch 1, wobei das nicht-verdauliche Oligosaccharid ferner Galacturonsäure-Oligosaccharide umfasst.

3. Verwendung nach Anspruch 1 oder 2, wobei die Zusammensetzung eine Ernährungszusammensetzung ist, wobei die Lipidkomponente 35 bis 50% der gesamten Kalorien liefert, die Proteinkomponente 7,5 bis 12,5% der gesamten Kalorien liefert und die Kohlenhydratkomponente 40 bis 55% der gesamten Kalorien liefert.

4. Verwendung nach einem der vorangehenden Ansprüche, wobei die Zusammensetzung an einen Säugling verabreicht wird.

5. Verwendung nach Anspruch 4, wobei der Säugling über einen Kaiserschnitt entbunden ist und/oder ein Frühgeborenes ist.

## Revendications

1. Utilisation d'une composition comprenant de oligosaccharide non-digestible, dans laquelle l'oligosaccharide non-digestible comprend des galacto-oligosaccharides et des fructo-oligosaccharides, pour la fabrication d'une composition destinée à prévenir une dégénérescence ou à améliorer une ou plusieurs aptitudes parmi comprenant (i) les aptitudes au langage, (ii) les aptitudes à la communication, (iii) les aptitudes sociales et/ou (iv) les aptitudes à la lecture.

2. Utilisation selon la revendication 1 dans laquelle l'oligosaccharide non-digestible comprend en outre des oligosaccharides d'acide galacturonique.

3. Utilisation selon les revendications 1 ou 2, dans laquelle la composition est une composition nutritionnelle dans laquelle l'élément lipidique apporte de 35 à 50 % du total des calories, l'élément protéique apporte de 7,5 à 12,5 % du total des calories et l'élément glucidique apporte de 40 à 55 % du total des calories.

4. Utilisation selon l'une quelconque des revendications précédentes, dans laquelle la composition est administrée à un nourrisson.

5. Utilisation selon la revendication 4, dans lequel le nourrisson est né par césarienne et/ou est un prématuré.
